# EUROPEAN PATENT APPLICATION

(11) **EP 4 691 551 A1**
(43) Date of publication of application: **11.02.2026**
(21) Application number: 23932173.0
(22) Date of filing: 07.04.2023
(51) Int. Cl.: A61N 5/10, H05H 3/06, H01J 27/10, G21K 1/12, G21K 1/02

(54) **NEUTRON BEAM GENERATION SYSTEM FOR NEUTRON CAPTURE THERAPY**

(30) Priority: 06.04.2023 KR 20230045522
(71) Applicant: Dawonmedax,. Ltd., Ansan-si Gyeonggi-do 15655 (KR)
(72) Inventor: HAN, Jae Eun, Siheung-si Gyeonggi-do 15022 (KR); KIM, Woong Hee, Incheon 21071 (KR); SEO, Hyeon Jin, Seoul 07023 (KR); KIM, Hyo Jin, Siheung-si Gyeonggi-do 14988 (KR); LEE, Soo Young, Siheung-si Gyeonggi-do 15011 (KR); KIM, Sung Hwan, Incheon 21632 (KR); PARK, Na Hyoung, Incheon 21632 (KR); WOO, Byeong Hyo, Ansan-si Gyeonggi-do 15457 (KR); YUN, Hyoung Jin, Siheung-si Gyeonggi-do 15011 (KR); KIM, Dong Su, Siheung-si Gyeonggi-do 15010 (KR); BAE, Young Soon, Incheon 22000 (KR); PARK, Sun Soon, Suwon-si Gyeonggi-do 16512 (KR)
(74) Representative: impuls legal PartG mbB
(86) International application number: PCT/KR2023/004714
(87) International publication number: WO 2024/210238

(57) **Abstract**

Disclosed is a neutron beam generation system for neutron capture therapy. According to an embodiment of the present disclosure, provided is a neutron beam generation system comprising: an injector which generates proton beams; an RF linear accelerator connected to the injector to accelerate the proton beams; a beamline which transmits the proton beams accelerated by the RF linear accelerator; a target which generates fast neutrons by colliding with the proton beams transmitted by the beamline; and a beam shaping assembly provided on one side of the beamline and configured to decelerate the fast neutrons into epithermal neutrons.

## Description

### Cross-Reference to Related Application

The present application is a continuation of International Application No. PCT/KR2023/004714, filed on April 7, 2023, which is based upon and claims priority to Korean Patent Application No. 10-2023-0045522, filed on April 6, 2023 in Korea. The entire disclosure of the above application is incorporated herein by reference.

### Technical Field

The present disclosure relates to a neutron beam generation system for neutron capture therapy.

### Background Art

The content described in this section merely provides background information for the present disclosure, but does not constitute the prior art.

Neutron Capture Therapy (NCT) refers to a method of treating cancer by injecting a boron component that captures or absorbs neutrons well, into cancer cells in the human body, irradiating the cancer cells with neutrons, and inducing the death of the cancer cells with strong energy generated by the nuclear reaction between boron and neutrons.

For neutron capture therapy, a device or system for generating and irradiating neutrons is required, and there are two main methods for generating neutrons having a spectrum suitable for cancer therapy. The first method is a method for generating neutrons by uranium fission generated in a nuclear reactor. Although studies on neutron generation by uranium fission have been conducted in several countries, there is a problem in that it is not suitable for hospital facilities for treating patients due to issues related to nuclear regulatory approvals and the like. The second method is a method for generating neutrons by colliding accelerated protons on a target composed of a material such as lithium or beryllium. The method for generating neutron by colliding a target by utilizing a proton accelerator is also suitable for a hospital facility for treating patients, and has been studied worldwide.

In order for a neutron generation device that uses proton acceleration and target collisions to be used in hospital facility, it must be safe from radiation and capable of continuously supplying epithermal neutron at a flux of 1×10⁹ neutrons/cm²·sec or more for 1 hour or more. In relation to this, there are problems such as energy loss during proton beam acceleration, the need for shielding due to radiation generation, excessive device size, device failures resulting from handling high-energy protons and neutrons, the need for long-lasting targets, and inefficient treatment due to long target replacement times.

### Summary

According to an embodiment, the neutron beam generation system includes an RF linear accelerator comprising a low-energy accelerating tube and a high-energy accelerating tube, thereby having a compact size and being capable of continuously generating high-quality epithermal neutrons at a flux of 1×10⁹ neutrons/cm²·sec or more.

According to an embodiment, a neutron beam generation system includes a beam direction adjusting unit comprising a bending magnet or the like, thereby enabling efficient treatment of patients by using a plurality of beamlines and a plurality of targets.

The problems to be solved by the present invention are not limited to the problems mentioned above, and other problems not mentioned will be clearly understood by those of ordinary skill in the art from the following descriptions.

According to an embodiment of the present disclosure, provided is a neutron beam generation system comprising: an injector which generates proton beams; an RF linear accelerator connected to the injector to accelerate the proton beams; a beamline which transmits the proton beams accelerated by the RF linear accelerator; a target which generates fast neutrons by colliding with the proton beams transmitted by the beamline; and a beam shaping assembly provided on one side of the beamline and configured to decelerate the fast neutrons into epithermal neutrons.

According to an embodiment, the neutron beam generation system has the effect of including an RF linear accelerator comprising a low-energy accelerating tube and a high-energy accelerating tube, thereby having a compact size and being capable of continuously generating high-quality epithermal neutrons at a flux of 1×10⁹ neutrons/cm²·sec or more.

According to an embodiment, the neutron beam generation system has the effect of including a beam direction adjusting unit comprising a bending magnet or the like, thereby enabling efficient treatment of patients by using a plurality of beamlines and a plurality of targets.

### Brief Description of the Drawings

FIG. 1 is a block diagram of a neutron beam generation system according to an embodiment of the present disclosure.
FIG. 2 is a perspective view of a neutron beam generation system according to an embodiment of the present disclosure.
FIG. 3 is a cross-sectional view of an injector according to an embodiment of the present disclosure.
FIG. 4 is a cross-sectional view of a low-energy accelerating tube according to an embodiment of the present disclosure.
FIG. 5 is a cross-sectional view of a high-energy accelerating tube according to an embodiment of the present disclosure.
FIG. 6 is a side cross-sectional view of a beamline and a target according to an embodiment of the present disclosure.
FIG. 7 is a top cross-sectional view of a beamline and a target according to an embodiment of the present disclosure.

### [Description of Signs]

110 Injector
120 RF linear accelerator
130 Beamline
140 Beam direction adjusting unit
150 Beam scanning unit
160 Target
170 Beam shaping assembly

### Detailed Description

Hereinafter, some embodiments of the present disclosure will be described in detail with reference to illustrative drawings. It should be noted that, when components in each drawing are denoted by reference numerals, the same components are denoted by the same numerals as much as possible even if they are shown in different drawings. In addition, in describing the present disclosure, when it is determined that a specific description of a related known configuration or function may obscure the subject matter of the present disclosure, the detailed description thereof will be omitted.

In describing the components of the present invention, terms such as first, second, A, B, (a), and (b) may be used. These terms are only used to distinguish the components from other components, and the nature, sequence, order, or the like of the components is not limited by the terms.

When a certain component is described as being "connected", "coupled", or "joined" to another component, it will be understood that the component may be directly connected or joined to the other component, but may also be "connected", "coupled", or "joined" via another component interposed there between.

Throughout the specification, when a part is said to "include" or "have" a component, this does not mean that it excludes other components, but rather that it may include other components, unless otherwise specifically stated.

The terms such as "unit" or "module" described in the specification refer to a unit that processes at least one function or operation, and may be implemented in hardware, software, or a combination thereof.

Unless otherwise specified, it should be understood that the description of any one embodiment may be applied to other embodiments as well.

The description of the invention disclosed below in connection with the appended drawings is intended to describe exemplary embodiments of the invention and is not intended to represent the only embodiments in which the invention may be practiced.

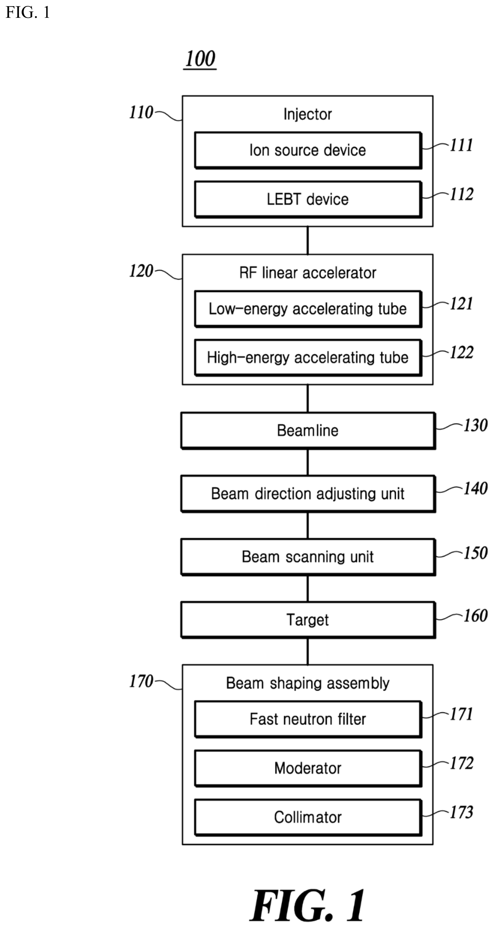

FIG. 1 is a block diagram of a neutron beam generation system according to an embodiment of the present disclosure.

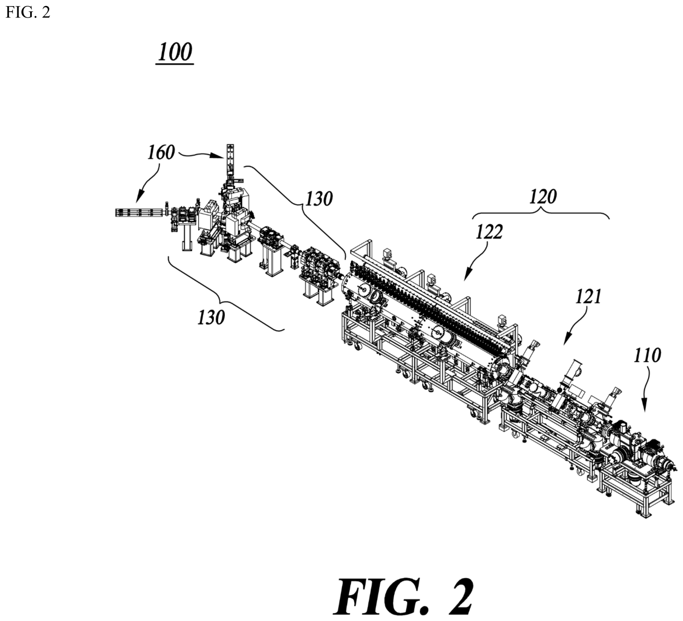

FIG. 2 is a perspective view of a neutron beam generation system according to an embodiment of the present disclosure.

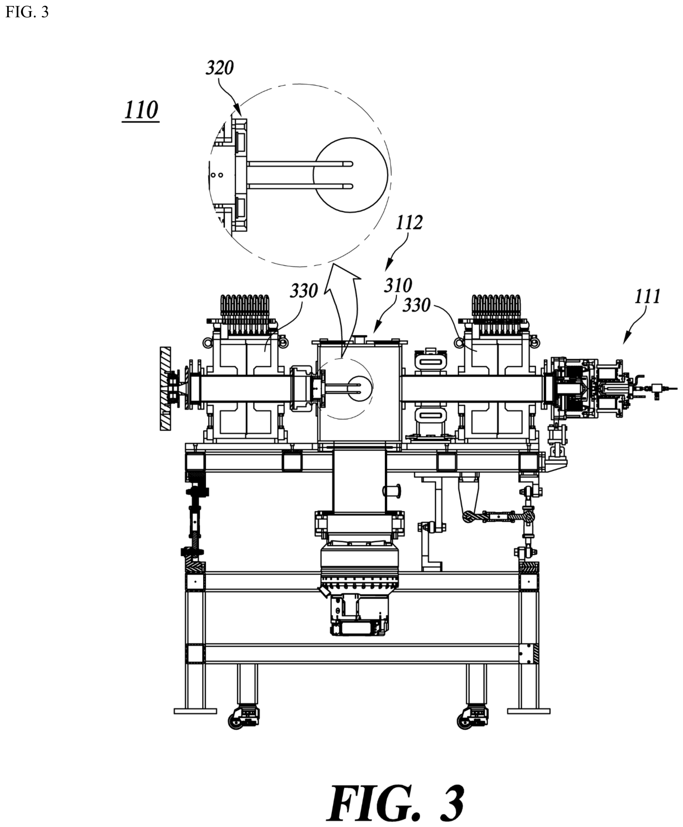

FIG. 3 is a cross-sectional view of an injector according to an embodiment of the present disclosure.

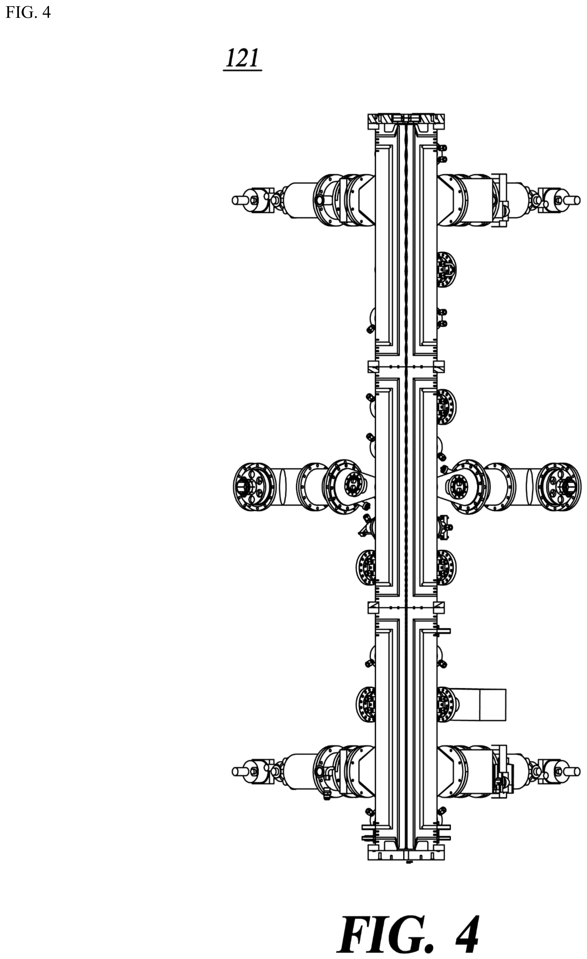

FIG. 4 is a cross-sectional view of a low-energy accelerating tube according to an embodiment of the present disclosure.

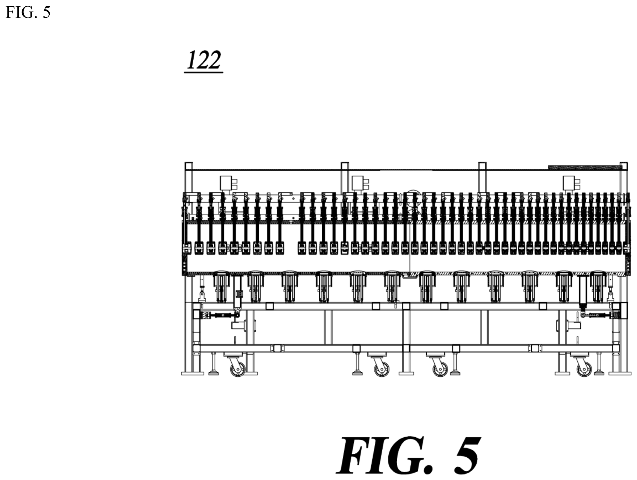

FIG. 5 is a cross-sectional view of a high-energy accelerating tube according to an embodiment of the present disclosure.

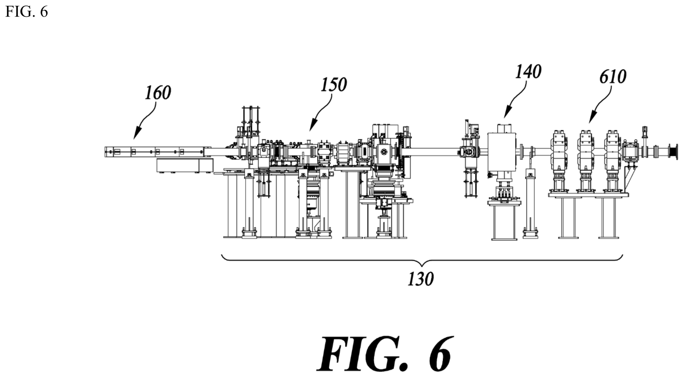

FIG. 6 is a side cross-sectional view of a beamline and a target according to an embodiment of the present disclosure.

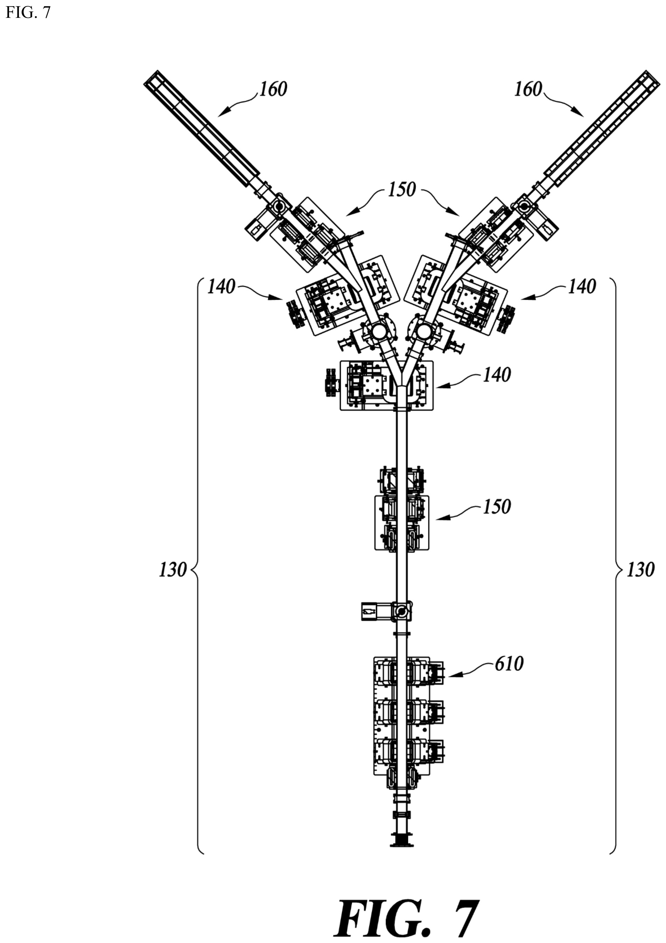

FIG. 7 is a top cross-sectional view of a beamline and a target according to an embodiment of the present disclosure.

Referring to FIGS. 1 to 7, a neutron beam generation system 100 according to an embodiment of the present disclosure may include an injector 110, a RF linear accelerator (radio frequency linear accelerator) 120, a beamline 130, a beam direction adjusting unit (beam direction adjustment unit) 140, a beam scanning unit 150, a target 160, a beam shaping assembly (BSA) 170, and a low level radio frequency (LLRF) device (not shown) for radio frequency (RF) control.

The injector 110 may include an ion source device 111 and a low energy beam transport (LEBT) device 112.

The RF linear accelerator 120 may include a low-energy accelerating tube (low energy accelerator) 121 and a high-energy accelerating tube (high energy accelerator) 122.

The beam shaping assembly 170 may include a fast neutron filter 171, a moderator 172, and a collimator 173.

The injector 110 is configured to generate a proton beam. The injector 110 may be of an ion source type. The injector 110 may extract positive hydrogen ions using a potential difference. The injector 110 is connected to the RF linear accelerator 120. The injector 110 generates the proton beam and transmits the proton beam to the RF linear accelerator 120. The injector 110 may generate the proton beam at a high current (e.g., a peak current of 40 mA or more) by forming a high-density plasma environment. The injector 110 may accelerate and focus the proton beam using an electric field. The injector 110 may accelerate the proton beam to a beam energy of 50 keV or more.

The ion source device 111 generates the proton beam. The ion source device 111 may extract positive hydrogen ions using a potential difference (e.g., 50 kV). The ion source device 111 may generate the proton beam at a high current by forming a high-density plasma environment using a duoplasmatron. The ion source device 111 may form a pulsed plasma in order to stably generate the high-density plasma. The ion source device 111 may accelerate and focus the proton beam using the electric field. The ion source device 111 may be connected to the LEBT device 112. The ion source device 111 may transmit the generated proton beam to the LEBT device 112.

The LEBT device 112 is configured to transmit the proton beam generated by the ion source device 111, to the RF linear accelerator 120. The LEBT device 112 may be connected to the ion source device 111 and the RF linear accelerator 120. The LEBT device 112 may also be disposed between the ion source device 111 and the RF linear accelerator 120. The LEBT device 112 may include a chamber 310 for diagnosing the beam size and the like. Hydrogen gas is inevitably generated in the duoplasmatron of the ion source device 111, and it is necessary to keep the inside of the LEBT device 112, the RF linear accelerator 120, and the like close to a vacuum in order to stably supply a high current. To this end, the LEBT device 112 according to the present disclosure may further include a pump for drawing the hydrogen gas in the inside of the chamber 310 and the like. In addition, as shown in FIG. 3, an orifice structure 320 may be formed in the chamber 310 to minimize hydrogen gas other than protons transmitted from the LEBT device 112 to the RF linear accelerator 120. Accordingly, the degree of vacuum in the insides of the LEBT device 112 and the RF linear accelerator 120 and the like may be improved to stably transmit the proton beam and supply the high current.

The LEBT device 112 is configured to transmit only protons (H+) among various types of hydrogen gases (e.g., H+, H2+, H3+, etc.) to the RF linear accelerator 120. As an example, the LEBT device 112 may include a solenoid magnet 330. The LEBT device 112 may accelerate and focus only protons among various types of hydrogen gases by using the solenoid magnet 330, and transmit them to the RF linear accelerator 120, while deflecting the remaining toward a wall surface of a beam transport tube or the like. The LEBT device 112 may include a plurality of solenoid magnets 330. Accordingly, it is possible to efficiently focus the protons and separate H2+ and H3+.

The RF linear accelerator 120 is configured to accelerate the proton beam transmitted by the injector 110. The RF linear accelerator 120 may be connected to the LEBT device 112 of the injector 110. The RF linear accelerator 120 may be connected to the beamline 130 to transmit the accelerated proton beam to the beamline 130. The RF linear accelerator 120 according to the present disclosure may accelerate the proton beam such that a beam energy of 10 MeV or more and a beam power of 25 kW or more are achieved. When the RF linear accelerator 120 accelerates the proton beam to a beam energy of 10 MeV and a beam power of 25 kW, a supply current may be 25 mA. The RF linear accelerator 120 may include the low-energy accelerating tube 121 and the high-energy accelerating tube 122, and thus may be configured in a compact size while accelerating the proton beam to a beam energy of 10 MeV or more and a beam power of 25 kW or more.

The low-energy accelerating tube 121 is connected to the LEBT device 112 and is configured to accelerate the proton beam transmitted from the LEBT device 112. The low-energy accelerating tube 121 according to the present disclosure may be a radio frequency quadrupole (RFQ) accelerating tube. The low-energy accelerating tube 121 may be configured to accelerate and focus the proton beam using the electric field. When the proton beam is accelerated and focused using the electric field, the transmission rate may be lower than that in a case of using a magnetic field. However, since a low-energy proton beam requires an excessively strong magnetic field for acceleration and focusing, and thus it is difficult to accelerate and focus the proton beam with the magnetic field, the low-energy accelerating tube 121 is configured to accelerate and focus, by using the electric field, the low energy proton beam transmitted from the LEBT device 112 to a certain level of energy (e.g., 3 MeV). Conversely, when a proton beam of high energy (e.g., 3 MeV or more) is accelerated and focused using the electric field, breakdown may occur due to the strong electric field. Here, breakdown refers to a phenomenon in which electrostatic breakdown occurs and neutrons are not generated during that time. The low-energy accelerating tube 121 according to the present disclosure is designed such that the intensity of the electric field is lowered to prevent breakdown from occurring. Specifically, the low-energy accelerating tube 121 may be designed such that a frequency difference between a resonance frequency of a quadrupole mode and a resonance frequency of a dipole mode is large. When the difference in resonant frequency between the two modes is small, a metal rod (dipole stabilizer rod) is inserted into the low-energy accelerating tube 121 such as the RFQ, so that the difference in the resonant frequency is large. In this case, the electric field may be increased by the inserted metal rod. In addition, the pump may be disposed in the vicinity of an inlet of the low-energy accelerating tube 121 to improve the vacuum level inside the low-energy accelerating tube 121 by drawing and removing hydrogen gas generated by the duoplasmatron of the ion source device 111 before the hydrogen gas flows into the low-energy accelerating tube 121. As a result, the low-energy accelerating tube 121 according to the present disclosure may stably accelerate the proton beam transmitted from the LEBT device 112 to a beam energy of 2 MeV to 4 MeV, preferably 3 MeV.

The high-energy accelerating tube 122 is connected to the low-energy accelerating tube 121 and is configured to accelerate the proton beam transmitted from the low-energy accelerating tube 121. The high-energy accelerating tube 122 according to the present disclosure may be a drift tube linear accelerator (DTL). The high-energy accelerating tube 122 may be configured to focus the proton beam using the magnetic field. When the proton beam is focused using the magnetic field, the transmission rate is higher than that in a case of using the electric field. However, as described above, it is difficult to focus the low-energy proton beam using the magnetic field. Accordingly, the RF linear accelerator 120 according to the present disclosure has a technical feature that the proton beam may be stably and efficiently accelerated to a high-energy level (e.g., 10 MeV), by accelerating and focusing the low-energy proton beam to a certain energy level (e.g., 3 MeV) using the low-energy accelerating tube 121, and accelerating the proton beam accelerated to the certain energy level, with the electric field by using the high-energy accelerating tube 122 and focusing the proton beam to the magnetic field. The high-energy accelerating tube 122 according to the present disclosure may be designed through simulation or the like such that the transmission rate of the proton beam is close to 100%.

The beam direction adjusting unit 140 is configured to adjust the direction of the proton beam accelerated and focused by the RF linear accelerator 120. The beam direction adjusting unit 140 may be disposed between the RF linear accelerator 120 and the beamline 130. The beam direction adjusting unit 140 may be disposed on the beamline 130. For example, as shown in FIGS. 6 and 7, when the neutron beam generation system 100 according to the present disclosure includes a triplet 610 for focusing the proton beam and the beam scanning unit 150 for scanning the proton beam at a high speed, the beam direction adjusting unit 140 may be disposed between the triplet 610 and the beam scanning unit 150 on the beamline 130. However, it should be noted that the position of the beam direction adjusting unit 140 is not limited to the above, and may be disposed at other positions as appropriate depending on the situation. The beam direction adjusting unit 140 may include a bending magnet. According to an embodiment of the present disclosure, the beam direction adjusting unit 140 is configured to adjust the direction of the proton beam, and includes a plurality of beamlines 130, a plurality of targets 160, and a plurality of beam shaping assemblies 170, thereby solving the problem that the treatment of the patient is not efficient due to a long target replacement time (which takes about 2-3 days for radiation cool down), and enabling effective treatment of the patient.

The beamline 130 is disposed between the RF linear accelerator 120 and the target 160 and is connected with the RF linear accelerator 120 and the target 160. The beamline 130 is configured to transmit the proton beam accelerated by the RF linear accelerator 120, to the target. The triplet 610 for focusing the proton beam may be disposed on the beamline 130 so that the proton beam transmitted along the length (e.g., 8 m) direction of the beamline 130 is stably transmitted to the target without dispersion. The triplet 610 may be configured such that a plurality of lenses (e.g., convex lenses, concave lenses) are sequentially disposed along a traveling direction of the proton beam. In addition, when the proton beam intensively collides with a narrow area of the target 160, the lifespan of the target 160 may be reduced. Therefore, by scanning the proton beam at a high speed to increase the area of the target 160 on which the proton beam collides, the lifespan of the target 160 may be improved. For this purpose, the beam scanning unit 150 may be disposed on the beamline 130. The beam scanning unit 150 may include a scanning magnet. As described above, the beam direction adjusting unit 140 may be disposed between the beam scanning unit 150 and the triplet 610.

The target 160 generates fast neutrons by colliding with the proton beam that has been accelerated by the RF linear accelerator 120 and transmitted via the beamline 130. The target 160 may be disposed on one side of the beamline 130. The target 160 may be disposed to be surrounded by the beam shaping assembly 170. The target 160 may be made of lithium, beryllium, or the like that smoothly generates neutrons by collision with the proton beam. However, when the target 160 is made of lithium, there is problems in radiation and stability. When the target 160 is made of beryllium, stability is ensured, but high beam energy is required to generate neutrons by colliding the proton beam with the beryllium target 160. Since the neutron beam generation system 100 according to the present disclosure may stably accelerate the proton beam to a beam energy of 10 MeV by using the RF linear accelerator 120 including the low-energy accelerating tube 121 and the high-energy accelerating tube 122, neutrons may be stably generated by forming the target 160 of beryllium.

The beam shaping assembly 170 may be disposed on one side of the beamline 130. The beam shaping assembly 170 may be disposed at a terminal end of one side of the beamline 130. The beam shaping assembly 170 is configured to decelerate fast neutrons generated by the collision of the proton beam with the target 160, into epithermal neutrons.

The beam shaping assembly 170 according to the present disclosure may be configured to have a conversion efficiency of 5×10⁷ epithermal neutrons/kW or more. Here, the conversion efficiency may refer to the number of epithermal neutrons generated per beam power (kW) of the proton beam. For example, it may be seen that the neutron beam generation system 100 according to the present disclosure may accelerate the proton beam to a beam power of 25 kW, and when the conversion efficiency of the beam shaping assembly 170 is 5×10⁷ epithermal neutrons/kW, the number of generated epithermal neutrons is 1.25×10⁹, which exceeds the 1×10⁹ epithermal neutrons required for effective neutron capture therapy, thereby it is suitable for neutron capture therapy.

The beam shaping assembly 170 may include the fast neutron filter 171, the moderator 172 disposed in front of the target 160 to reduce the energy of neutrons scattered in the incident direction of the proton beam, the collimator 173 configured to collect the neutrons to an affected part of the patient, and a reflector (not shown) configured to accommodate the moderator 172 and the collimator 173 and shield external leakage of the epithermal neutrons and gamma rays. The beam shaping assembly 170 may further include a neutron reflecting device (not shown) disposed outside the terminal end of the beamline 130 and behind the target 160, and configured to reflect neutrons scattered rearward from the target 160 back to the front. The neutron reflecting device may be made of a material such as lead having a property of reflecting neutrons scattered rearward from the target 160. By including the neutron reflecting device, the beam shaping assembly 170 according to the present disclosure may reflect neutrons scattered and lost rearward from the target 160 back toward the target, thereby implementing an improved flux of epithermal neutrons.

Various implementations of the systems and techniques described in the present specification may be realized in digital electronic circuits, integrated circuits, field programmable gate arrays (FPGAs), application specific integrated circuits (ASICs), computer hardware, firmware, software, and/or combinations thereof. These various implementations may include implementations in one or more computer programs executable on a programmable system. The programmable system includes at least one programmable processor (which may be a special-purpose processor or a general-purpose processor) coupled to receive data and instructions from, and to transmit data and instructions to, a storage system, at least one input device, and at least one output device. Computer programs (also known as programs, software, software applications, or code) include instructions for the programmable processor and are stored on a "computer-readable recording medium".

The computer-readable recording medium includes all kinds of recording devices in which data readable by a computer system is stored. The computer-readable recording medium may be a non-volatile or non-transitory medium such as a ROM, a CD-ROM, a magnetic tape, a floppy disk, a memory card, a hard disk, a magneto-optical disk, or a storage device, and may further include a transitory medium such as a data transmission medium. In addition, the computer-readable recording media may be distributed in a network-connected computer system, and computer-readable codes may be stored and executed in a distributed manner.

Various implementations of the systems and techniques described in the present specification may be implemented by a programmable computer. Here, the computer includes a programmable processor, a data storage system (which include volatile memory, non-volatile memory, or another type of storage system, or combinations thereof), and at least one communication interface. For example, the programmable computer may be one of a server, a network device, a set-top box, an embedded device, a computer expansion module, a personal computer, a laptop, a personal data assistant (PDA), a cloud computing system, or a mobile device.

The above description is merely illustrative of the technical idea of the present embodiments, and various modifications and variations will be possible to those skilled in the art without departing from the essential characteristics of the present embodiment. Therefore, the present embodiments are intended to be illustrative rather than limiting of the technical idea of the present embodiments, and the scope of the technical idea of the present embodiments is not limited by these embodiments. The protection scope of the present embodiment should be interpreted by the following claims, and all technical ideas falling within the scope equivalent thereto should be interpreted as being included in the scope of rights of the present embodiments.

## Claims

1. A neutron beam generation system comprising:
an injector which generates proton beams;
an RF linear accelerator connected to the injector to accelerate the proton beams;
a beamline which transmits the proton beams accelerated by the RF linear accelerator;
a target which generates fast neutrons by colliding with the proton beams transmitted by the beamline; and
a beam shaping assembly provided on one side of the beamline and configured to decelerate the fast neutrons into epithermal neutrons.

2. The neutron beam generation system of claim 1, wherein the system is configured to generate the epithermal neutrons at a flux of 1×10⁹ neutrons/cm²·sec or more.

3. The neutron beam generation system of claim 2, wherein the system is configured to continuously generate the epithermal neutrons for 40 minutes or more.

4. The neutron beam generation system of claim 1, wherein the injector comprises:
an ion source device for generating the proton beam; and
a low energy beam transport (LEBT) device for transmitting the proton beam to the RF linear accelerator.

5. The neutron beam generation system of claim 3, wherein the injector is of an ion source type.

6. The neutron beam generation system of claim 4, wherein the ion source device is configured to generate the proton beam using a potential difference.

7. The neutron beam generation system of claim 6, wherein the ion source device is configured to generate positive hydrogen ions.

8. The neutron beam generation system of claim 4, wherein the ion source device is configured to accelerate and focus the proton beam using an electric field.

9. The neutron beam generation system of claim 8, wherein the ion source device accelerates the proton beam to an energy of up to 50 keV.

10. The neutron beam generation system of claim 4, wherein the ion source device generates the proton beam using a plasma environment.

11. The neutron beam generation system of claim 10, wherein the ion source device forms the plasma environment using a duoplasmatron.

12. The neutron beam generation system of claim 10, wherein the ion source device generates the proton beam at a peak current of 40 mA or more.

13. The neutron beam generation system of claim 10, wherein the ion source device forms a pulsed plasma.

14. The neutron beam generation system of claim 4, wherein the LEBT device comprises a solenoid magnet.

15. The neutron beam generation system of claim 14, wherein:
a plurality of the solenoid magnets are provided.

16. The neutron beam generation system of claim 4, wherein the LEBT device comprises:
a chamber; and
one or more pumps connected to the chamber.

17. The neutron beam generation system of claim 16, wherein the chamber comprises an orifice structure.

18. The neutron beam generation system of claim 1, wherein the RF linear accelerator accelerates the proton beam such that an energy of the proton beam is 10 MeV or more.

19. The neutron beam generation system of claim 18, wherein:
a power of the proton beam is 25 kW or more.

20. The neutron beam generation system of claim 1, wherein the RF linear accelerator comprises a low-energy accelerating tube and a high-energy accelerating tube.

21. The neutron beam generation system of claim 20, wherein the low-energy accelerating tube is an RFQ (Radio Frequency Quadrupole), and the high-energy accelerating tube is a DTL (Drift Tube LINAC(Linear Accelerator)).

22. The neutron beam generation system of claim 20, wherein the low-energy accelerating tube accelerates the proton beams to a beam energy of 3 MeV or less.

23. The neutron beam generation system of claim 22, wherein the high-energy accelerating tube accelerates the proton beam accelerated by the low-energy accelerating tube, to a beam energy of 10 MeV or more.

24. The neutron beam generation system of claim 20, wherein the low-energy accelerating tube is configured to accelerate and focus the proton beam using an electric field.

25. The neutron beam generation system of claim 20, wherein the high-energy accelerating tube is configured to accelerate and focus the proton beam using a magnetic field.

26. The neutron beam generation system of claim 20, wherein the low-energy accelerating tube is designed such that a frequency difference between a resonance frequency of a quadrupole mode and a resonance frequency of a dipole mode is large.

27. The neutron beam generation system of claim 20, wherein one or more pumps are disposed in the low-energy accelerating tube.

28. The neutron beam generation system of claim 1, further comprising:
a beam direction adjusting unit configured to adjust a direction of the proton beam.

29. The neutron beam generation system of claim 28, wherein the beam direction adjusting unit comprises one or more bending magnets.

30. The neutron beam generation system of claim 28, wherein the beamline, the target, and the beam shaping assembly are provided in plurality.

31. The neutron beam generation system of claim 1, wherein:
a triplet configured to focus the proton beam is disposed on the beamline.

32. The neutron beam generation system of claim 31, wherein the triplet comprises one or more lenses.

33. The neutron beam generation system of claim 32, wherein the one or more lenses are sequentially disposed along a traveling direction of the proton beam.

34. The neutron beam generation system of claim 1, further comprising:
a beam scanning unit disposed on the beamline and configured to scan the proton beam.

35. The neutron beam generation system of claim 1, wherein the target is made of beryllium.

36. The neutron beam generation system of claim 1, wherein:
a conversion efficiency of the beam shaping assembly is 5×10⁷ epithermal neutrons/kW or more.

37. The neutron beam generation system of claim 1, wherein the beam shaping assembly comprises a neutron reflector that reflects neutrons scattered rearward of the target toward the target.

38. The neutron beam generation system of claim 37, wherein at least a portion of the neutron reflector is made of lead.

39. The neutron beam generation system of claim 1, wherein the beam shaping assembly is disposed at a terminal end of the beamline and is configured to receive the target.

40. The neutron beam generation system of claim 1, wherein the beam shaping assembly comprises:
a moderator disposed in front of the target and configured to reduce energy of the fast neutrons scattered in an incident direction of the proton beam;
a collimator configured to collect the epithermal neutrons to an affected part of a patient; and
a reflector configured to accommodate the moderator and the collimator and shield external leakage of the epithermal neutrons and gamma rays.
